Europäisches Patentamt
European Patent Office
Office européen des brevets

(11) Publication number: **0 054 576 B2**

# NEW EUROPEAN PATENT SPECIFICATION

(45) Date of publication of the new patent specification: **13.07.88**

(51) Int. Cl.[4]: **C 07 C 67/04**, B 01 J 31/08, C 07 C 69/14, C 07 C 69/24

(21) Application number: **80108032.6**

(22) Date of filing: **18.12.80**

(54) **Process for producing esters from olefins.**

(43) Date of publication of application: **30.06.82 Bulletin 82/26**

(45) Publication of the grant of the patent: **21.03.84 Bulletin 84/12**

(45) Mention of the opposition decision: **13.07.88 Bulletin 88/28**

(84) Designated Contracting States: **DE FR GB IT NL**

(56) References cited:
**DE-A-2 306 586**
**DE-A-2 602 856**
**DE-B-1 168 908**
**GB-A-2 041 364**
**US-A-3 053 887**
**US-A-3 922 294**

**MERCK INDEX page 1581**
**ULLMANN'S Encyclopädie der technischen Chemie, 4th Ed., Vol. 3, pp. 500-501, 514**

**The file contains technical information submitted after the application was filed and not included in this specification**

(73) Proprietor: **UNION EXPLOSIVOS RIO TINTO, S.A.**
**Marqués del Riscal, 2**
**Madrid-4 (ES)**

(72) Inventor: **Gadea, Jesus Santa-Olalla**
**Plaza de America Edif. Torre de America**
**12th Floor Huelva (ES)**

(74) Representative: **Vossius & Partner**
**Siebertstrasse 4 P.O. Box 86 07 67**
**D-8000 München 86 (DE)**

EP 0 054 576 B2

Courier Press, Leamington Spa, England.

Process for producing esters from olefins

This invention relates to a process for the continuous production of organic esters by the reaction of carboxylic acids with olefins in the presence of a cationic exchange resin type of catalyst.

More particularly, this invention relates to a process for reacting lower carboxylic acids, especially acetic acid or propionic acid, with lower olefins, especially propylene or butylene, in a fixed-bed tubular flow reactor in which the acid in the liquid state is continuously contacted with the olefin, which is present as a gas. The solid catalyst used is an ion exchange resin, preferably in its acid form.

The production of esters by means of the reaction of a carboxylic acid with an olefin in the presence of a strong acid as catalyst has been known for several years. Over the last few years a number of catalysts have been used, usually strong mineral acids such as sulphuric acid, perchloric acid, or even less strong acids such as phosphoric acid, all of which act by themselves as homogeneous catalysts or else as supported heterogeneous catalysts on inert materials.

For example, from US patent 2,740,800 there is known a process which comprises continuously feeding acetic acid and propylene into a bath of approximately the following composition (in percent by weight):
20—30% sulphuric acid, 5—30% acetic acid, 5—10% isopropyl acetate, 0.5—10% water and 20—69.5% isopropyl hydrogen sulphate, maintaining said bath at a temperature of about 60—80°C, the rate of feed of said propylene being about 2—5 cubic metres per hour of propylene per kg. of said bath, the rate of feed of said acetic acid being sufficient to maintain the bath content thereof within the above-mentioned percentage range, and removing isopropyl acetate from the bath by gas containing propylene at substantially the rate at which the isopropyl acetate is formed in the bath.

There is further known from DE—OS 25 11 978 a process for preparing carboxylic acid esters of the formula I

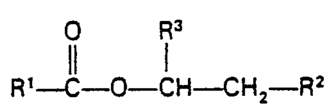 (I)

wherein $R^1$ and $R^2$ are the same or different and each mean an aliphatic, cycloaliphatic, araliphatic or aromatic group or a hydrogen atom, $R^3$ designates a hydrogen atom or $R^3$ and $R^2$ stand for members of an alicyclic ring, by reacting carboxylic acids with olefins in the presence of phosphoric acid as catalyst, characterised by reacting carboxylic acids of the formula II

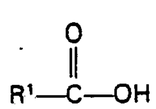 (II)

wherein $R^1$ has the meaning given above, with olefins of the formula III

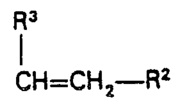 (III)

wherein $R^2$ has the meaning given above, at a temperature of 141 to 350°C in the presence of a catalyst containing silicium dioxide and phosphoric acid in an amount of 10 to 70 percent by weight phosphoric acid, based on the total amount of catalyst, and in the presence of phosphoric acid and/or one or more phosphoric acid esters as auxiliary catalysts.

From EP A1 0 003 206 there is known a process for the preparation of ethyl or isopropyl esters by reaction in liquid phase of ethylene or propylene, under pressure, with a carboxylic acid, characterised in that the reaction is carried out in the presence of an effective amount of at least one acid of the formula

$$C_nF_{2_{n+1}}SO_3H$$

in which n is an integer which can be zero and which has at most the value 8, as a catalyst.

Direct esterification of organic acids with olefins in the presence of strong acids in the homogeneous phase as catalysts according to the above-mentioned prior art presents the drawback that the resulting esterification product has to be separated from the catalyst in a laborious and uneconomical fashion. If, for example, sulphuric acid is used as the catalyst, it can be washed out of the organic reaction products with water. It is, however, uneconomical to recover the sulphuric acid by concentration. It is likewise uneconomical to discard the sulphuric acid since it contains part of the organic acid used.

In addition to the use of various acids as catalyst in the homogeneous phase which has already been mentioned, there are also known methods in which ion exchange resins are used as catalysts. US patent 2,678,332 describes a process for the esterification of olefins with organic acids in which ion exchange resins are used as catalysts.

After the esterification reaction is complete, the ester-acetic acid mixture can be directly decanted from the solid resin particles or filtered from them and thereafter worked up in the usual way with a convenient method for the recovery of unreacted acetic acid or other water-soluble organic material present in the reaction products.

According to the processes known from US patent 2,678,332, however, the reaction between the carboxylic acid and the olefin is carried out in the liquid phase. In order to ensure that the reaction partners are sufficiently well mixed the ingredients have to be vigorously stirred. When an olefin is used which is gaseous at the reaction

temperature, sufficient pressure must be exerted on the reactor to maintain the olefin in a liquid phase at the operating temperature. Such pressures range from slightly above atmospheric pressure up to about 55 atmospheres. The chief purpose for the use of such elevated pressures is to maintain the olefin as well as any other low-boiling reactants in the liquid phase.

It can be stated that these conventional reaction systems are constituted by two phases: the catalyst solid phase and the olefin-carboxylic acid mixture liquid phase. In order to keep the olefin in the liquid form, when dealing with gas olefins under the temperature conditions required by the reaction very high pressures are needed which are badly tolerated by the catalyst packing. The consequence is a drastic reduction in the catalytic life. Furthermore, the reactions are carried out using vertical tubular flow reactors, where the pressurized liquid olefin is fed through the top of the reactor after being mixed up with the liquid carboxylic acid and comes into contact with the solid catalyst packed bed while flowing downwards through it. This can lead to a poor stirring effect. Good stirring is a rate determining factor and consequently the yield-determining factor of the process. As a consequence of these facts the ester yields in the process known from US patent 2,678,332 are very low.

Finally there is known from US patent 3,922,294 a process for the manufacture of isopropyl esters by reacting propylenes with carboxylic acids in trickling phase, which comprises the step of passing gaseous propylene and an aliphatic, cycloaliphatic, or aromatic carboxylic acid having up to 20 carbon atoms either in liquid form or in the form of a solution in an inert solvent continuously in parallel flow over an acid ion exchanger of the sulfonic acid type at a temperature in the range of from 40° to 170°C.

In a system with downward parallel flow of the reactants (liquid droplets trickle downward) complete wetting of the catalyst is doubtful, there is no stirring effect and the heat transfer is unsatisfactory. As a consequence the catalyst is subject to temperature and drying-up mechanical strain resulting in poor catalyst life and poor space-time yield.

It is an object of the present invention to provide a process for the continuous production of carboxylic acid esters which overcomes the disadvantages of the prior processes.

It is a further object of the present invention to provide a simple and efficient process for the continuous production of carboxylic acid esters in a high yield.

It is a further object of the present invention to provide a process for the continuous production of carboxylic acid esters in a very high selectivity.

These and other objects of the present invention will become evident from the following description and the accompanying drawings.

The invention accordingly comprises a process for the continuous production of carboxylic acid esters having the general chemical formula I

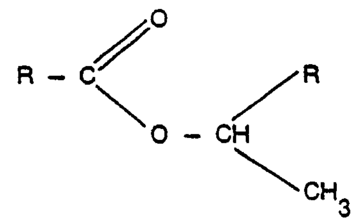

(I)

wherein R is a methyl or ethyl group, by direct reaction of either acetic or propionic acid with either propylene or butylenes in the presence of a cation exchange resin which is based on a styrene-divinylbenzene copolymer and has a divinylbenzene content of from 8 to 18% and a particle size between 0.3 and 1.5 mm, as a catalyst, wherein the olefin in its gaseous state (gas phase) and the carboxylic acid as the liquid phase are passed in parallel flow through a vertical reaction tube containing the catalyst, which process is characterized in that

a) before starting the reaction the catalyst is subjected to a dehydration treatment followed by an immersion treatment in the carboxylic acid;

b) the liquid carboxylic acid and the gaseous olefin are then fed concurrently through the bottom of the reaction vessel, the catalyst during the whole reaction being completely flooded with the carboxylic acid; and

c) the reaction is carried out, when the olefin used is propylene, at a pressure of 6 to 20 kg/cm$^2$, and when the olefin is butylene, at a pressure of about 3.5 kg/cm$^2$, with reaction temperatures between 70 and 115°C.

The reaction between the carboxylic acid and the olefin is carried out at a pressure which is not, however, sufficient to liquefy the olefin at the given reaction temperature.

In contrast to the known processes, where the olefin is liquefied due to pressure and the reaction mixture therefore only consists of two phases (solid catalyst and liquid reaction mixture), the reaction system according to the present invention is a three-phase chemical system. The olefin is fed and flows through the reactor as a gas and becomes dissolved into the liquid carboxylic acid feed to the extent required by the reaction. Thereby the contact between the liquid olefin-carboxylic acid solution and the solid catalyst bed particles is improved by the stirring effect of the gaseous olefin flow.

A three-phase reaction system of this type, with a stationary solid catalyst phase and a mobile liquid and mobile gaseous phase is normally operated in countercurrent; see for example US patent 3,053,887. Thereby the gas is fed through the bottom of the vertical tubular reactor and as it flows upwards gets into countercurrent contact with the downward flowing liquid acid. In this technique there is, however, a danger that the catalyst bed may not always be completely covered by the liquid phase. Locally gas bubbles and flow discontinuities can form, which are undesirable for a good contact between the gas-liquid reactants and the catalyst. These phenomena will also be detrimental to the

activity, selectivity and life of the catalyst.

Surprisingly it was found that in the continuous production of carboxylic acid esters according to the process of the present invention particularly good results are obtained as regards yield and selectivity when gas and liquid are fed concurrently through the bottom of the vertical tubular reactor. This way of feeding the liquid and the gaseous reaction partners is quite atypical for reactions in a three-phase system, since as a rule reactions of this type are carried out in countercurrent. A person skilled in the art could not, therefore, expect that in the process according to the invention the unconventional technique used would lead to especially good results.

In the case of the inventive manner of feeding the liquid and gaseous reaction partners in direct current, the whole catalyst bed is kept flooded with the liquid carboxylic acid from the start of the reaction. Contact of the gas bubbles and catalyst particles is thus occasional and kept to a minimum in space and time throughout the reactor. On the other hand, the liquid flow regime through the catalyst particles is made turbulent by the stirring effect of the gas flow. In the present process pressure levels of 6 to 12 atm. — preferably 8 atm. — are enough to make the contact of propylene and acetic acid with the active surface of the catalyst highly efficient and to ensure that propylene concentration in the liquid phase and in the liquid-solid chemically active catalyst interphase is high enough. These pressure levels are quite different from those which would be required for a conventional liquid phase system in order to keep the olefin in liquid form. For example, 40 atmospheres are needed for propylene at 85°C.

In comparison to the known processes, the process of this invention offers the following advantages:

A) Thanks to the defined conditions of the introduction of carboxylic acid and olefin in direct current from the bottom of reactor it is ensured that the catalyst is always flooded with liquid and that no sizeable gas bubbles form which could lead to partial desiccation of the catalyst. The result is a high ester yield.

B) As compared to liquid homogeneous catalysts, the use of a solid heterogeneous catalyst means easier separation from the reactants and considerably less corrosion effects.

C) The need to completely separate the reactants from the catalyst no longer presents a problem. When mineral acids are used as catalysts, such separation has to be brought about by water washing or by distillation. Recovery of the acids by concentrating the wash water is not economical and it is not easy to treat them as effluents in view of the large amounts of acetic acid which are carried along.

These drawbacks are clearly overcome by the use of cation exchange resins in their acid form as catalysts, since reactants flow through the catalyst bed and out of it without dragging along any of the active catalyst ingredients.

The reaction product can be transferred directly to the purification stage, where the unreacted carboxylic acid is separated and recycled.

D) The process of the present invention, which is based on the three-phase reaction system and the use of exchange resins as catalysts, allows for reaction temperatures which are lower than those required in the esterification processes of organic acids with olefins in the vapour phase on supported acid catalysts. This produces a definite advantage in that by-product formation due to higher temperature levels is reduced considerably, which is equivalent to an increase in process selectivity.

Catalysts suitable for use in the process of this invention are cationic exchange resins, particularly strong acid cationic exchangers such as those of the sulfonic acid type which are, for example, commercially available under the "Amberlite" or "Levatite" trademarks.

Preferred are cationic exchange resins whose basic structure consists of a styrene-divinylbenzene macroporous copolymer. Experiments have been made with resins having a variable divinylbenzene content of between 8 and 18%, with particle sizes in the range of 0.3 to 1.5 mm and a water content ranging from 0.6 to 0.9%.

The most favourable results have been obtained with those resins in which the divinylbenzene content is closer to 8%. At higher divinylbenzene contents the elasticity of the particles is diminished and the osmotic shock makes fragmentation easier.

The ion exchange capacities of the catalysts, as measured by acid-base titration, oscillate between 4.0 and 5.5 H meq/g of dry resin.

Before starting the reaction process it is necessary to submit the catalyst to a dehydration treatment followed by an immersion treatment. The latter is carried out by keeping the catalyst bed submerged in a solvent similar to the reaction medium — for example acetic acid — for a period of time long enough to achieve the optimum swelling point.

The dehydration pretreatment of the catalyst ion exchange resin is conducted at a temperature of at least 100°C, preferably between 105 and 115°C, for at least ten hours, preferably at least twenty hours, until a residual humidity value of 0.6 to 0.9% and a dry resin density value of 0.86 to 0.87 g/ml are obtained. After the dehydration treatment the dry resins are immersed with the carboxylic acid which it is intended to react with the olefin up to an absorption value of at least 0.80, preferably at least 0.90 g of acid per g of selected dry resin.

The reactants are continuously fed to the reactor in an olefin to acid molar ratio of at least 1:1, as it is convenient to have an excess of olefin at all times. The preferred molar ratio of olefin to acid is in the range of from 1:1 to 2.5:1.

The liquid hourly space velocity (LHSV) of the acetic acid flow is in the range of from 2.2 to 1.0 $h^{-1}$, preferably of from 2.0 to 1.5 $h^{-1}$. The flows

are to be kept as constant as possible during the process in order to maintain good isothermicity in all parts of the catalytic reactor.

Bearing in mind what has been stated above in connection with the adequate pressure for a three-phase reaction system, pressure should be the highest compatible with these limitations because it must have the favourable effect to be expected from gas-liquid addition reactions. Pressures from atmospheric to 20 kg/cm$^2$ have been used. The best results are obtained within the 6 to 12 kg/cm$^2$ interval.

The exothermic heat of reaction makes good temperature control necessary. This control is made easier in the process of this invention by the fact that the tubular packed bed catalytic reactor is flooded all the time with the liquid flow of reactants. This results in more uniform heat distribution inside the reactor, to which the turbulence brought about by the gas flow is also a contributory factor.

The reaction has been carried out in the particular system of the invention over a wide range of temperatures. The most favourable results are obtained at temperatures between 70 and 115°C. The preferred temperature range is from 90 to 110°C. Higher temperatures than these increase the production of undesirable by-products such as propylene polymers to a very large extent. The result of this is not only the reduction of the reaction yield, but also the gradual deposition of these products on the catalyst surface, thereby reducing catalyst activity.

The process of the invention is illustrated further with the aid of the attached drawing.

In the drawing (1) means a feed tank for the olefin and (2) a feed tank for the carboxylic acid.

After dehydration and impregnation with the carboxylic acid the catalyst is charged into the multitubular reactor (3). The catalyst bed thus obtained is now flooded with the liquid carboxylic acid, taking care to ensure that the catalyst is fully covered. At the same time heating is begun.

When this operation is completed propylene can already be fed, which in turn serves to pressurize the reactor. The reaction is started and the reaction heat produced will help considerably to maintain an adequate thermal reaction level.

When the desired pressure is attained in the circuit, back pressure valves regulate the outlets of the liquid-vapor-separator (4) where excess propylene gas expands to atmospheric pressure, thus being separated from the organic liquid phase. This propylene is compressed and recycled to the feed tank (1).

The liquid phase is fed to the middle zone of the distillation tower (5). Here the light fraction is continuously separated.

The other fraction goes to a second tower (6) from the top of which pure isopropyl acetate is obtained. The residue from this tower is circulated to a third distillation tower (7) which effects the separation of unreacted acetic acid, which is recycled back to the feed tank (2), from the residual heavier by-products.

The following examples are intended to illustrate but in no way limit the scope of the present invention.

Example 1

17 g of ion exchange resin Lewatit SPC, with sulphonic acid groups, previously dehydrated at a temperature of 110°C for 25 hours and immersed in acetic acid until an absorption value of 0.95 g of acid per g of dry resin is reached are placed in a vertical stainless steel tube with a height of 50 cm and a diameter of 1.09 cm provided with a steam jacket and designed to withstand pressure. The catalyst bed is thus provided with an apparent volume of 42.9 cm$^3$ (volume occupied by the packed bed).

The reactor is flooded with liquid acetic acid flowing at 68 g/h and a LHSV of 1.75 h$^{-1}$. Finally 76.3 g/l of propylene gas at a pressure of 8 kg/cm$^2$ and a temperature of 95°C are continuously fed through.

After operating the system for 8 hours the reaction product collected was analysed with the following result:

| | |
|---|---|
| Acetic acid conversion | 71.8% |
| Propylene conversion | 46.3% |
| Isopropyl acetate selectivity | 96.5% |

Example 2

The reaction system of the preceding example was operated continuously for 136 hours, using the same temperature and pressure conditions. Propylene and acetic acid were continuously fed in a molar ratio of 2.1:1. Catalyst activity and selectivity remained constant during the whole period. The results were as follows:

| | |
|---|---|
| Acetic acid conversion | 70% |
| Propylene conversion | 34% |
| Isopropyl acetate selectivity | 97% |

Example 3

In order to check further the effect of the reactant flows and temperature on the activity, selectivity and life of the catalyst, the test of the preceding example was continued, feeding 67 g/h of acetic acid and a LHSC of 1.7 h$^{-1}$ and 95 g/h of propylene continuously over 160 hours. The reactor pressure was 8 kg/cm$^2$ and the temperature was kept steady at 108°C. Once again conversion and selectivity remained constant during the whole time, with the following values.

| | |
|---|---|
| Acetic acid conversion | 87% |
| Propylene conversion | 44% |
| Isopropyl acetate selectivity | 97% |

LEGEND OF THE FLOW SHEET

EQUIPMENTS:

1. — Feed tank (olefin).

2. — Feed tank (acetic acid).

3. — Catalytic Multitubular reactor.

4. — Vapor-liquid separator.

5. — Distillation tower.

6. — Distillation tower for isopropil acetate.

7. — Distillation tower for recovery acetic acid.

8. — Compressor.

**Claims**

1. A process for the continuous production of carboxylic acid esters having the general chemical formula I

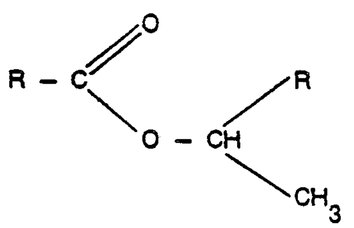 (I)

wherein R is a methyl or ethyl group, by direct chemical reaction of either acetic or propionic acid with either propylene or butylenes in the presence of a cationic exchange resin which is based on a styrene-divinylbenzene copolymer and has a divinylbenzene content of from 8 to 18% and a particle size between 0.3 and 1.5 mm, as a catalyst, wherein the olefin in its gaseous state (gas phase) and the carboxylic acid as the liquid phase are passed in parallel flow through a vertical reaction tube containing the catalyst, characterized in that

a) before starting the reaction the catalyst is subjected to a dehydration treatment followed by an immersion treatment in the carboxylic acid;

b) the liquid carboxylic acid and the gaseous olefin are then fed concurrently through the bottom of the reaction vessel the catalyst during the whole reaction being completely flooded with the carboxylic acid; and

c) the reaction is carried out, when the olefin used is propylene, at a pressure of 6 to 20 $kg/cm^2$, and when the olefin used is butylene, at a pressure of about 3.5 $kg/cm^2$, with reaction temperatures between 70 and 115°C.

2. A process according to Claim 1, characterized in that the carboxylic acid is fed in at a liquid hourly space velocity (LHSV) of from 2.0 to 1.5 $h^{-1}$.

3. A process according to Claim 2, characterized in that the carboxylic acid and the olefin are supplied in a molar ratio of 1:1 to 1:2.5.

**Patentansprüche**

1. Ein Verfahren zur kontinuierlichen Herstellung von Carbonsäureestern deer allgemeinen chemischen Formel I

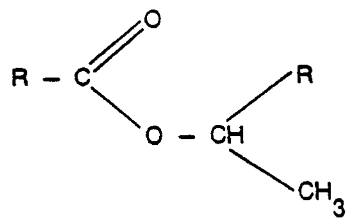 (I)

in der R eine Methyl- oder Äthylgruppe bedeutet, durch direkte chemische Umsetzung von entweder Essig- oder Propionsäure mit entweder Propylen oder Butylenen in Gegenwart eines Kationenaustauscherharzes, das auf einem Styrol-Divinylbenzol-Copolymerisat beruht und einen Divinylbenzol-Gehalt von 8 bis 18% und eine Teilchengröße zwischen 0,3 und 1,5 mm aufweist, als Katalysator, wobei das Olefin in gasförmigem Zustand (Gasphase) und die Carbonsäure als flüssige Phase in parallelem Flüß durch ein vertikales, den Katalysator enthaltendes Reaktionsrohr geführt werden, dadurch gekennzeichnet, daß

a) vor Beginn der Umsetzung der Katalysator einer Entwässerungsbehandlung und dann einer Eintauchbehandlung in der Carbonsäure unterzogen wird,

b) die flüssige Carbonsäure und das gasförmige Olefin dann im Gleichstrom durch den Boden des Reaktionsgefäßes eingespeist werden und wobei der Katalysator während der gesamten Umsetzung vollständig mit der Carbonsäure überschwemmt ist, und

c) die Umsetzung, wenn das verwendete Olefin Propylen ist, bei einem Druck von 6 bis 20 $kg/cm^2$, und wenn das verwendete Olefin Butylen ist, bei einem Druck von etwa 3,5 $kg/cm^2$, bei Reaktionstemperaturen zwischen 70 und 115°C durchgeführt wird.

2. Verfahren nach Anspruch 1, dadurch gekennzeichnet, daß die Carbonsäure mit einer Raumgeschwindigkeit (LHSV) von 2,0 bis 1,5 $Std.^{-1}$ eingespeist wird.

3. Verfahren nach Anspruch 2, dadurch gekennzeichnet, daß die Carbonsäure und das Olefin in einem Molverhältnis von 1:1 bis 1:2,5 zugeführt werden.

**Revendications**

1. Procédé pour la production en continu d'esters carboxyliques représentés par la formule chimique générale (I)

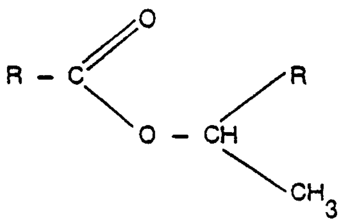 (I)

dans laquelle R est un groupe méthyle ou éthyle, par la réaction directe de l'acide acétique ou propionique avec soit du propylène, soit des butylènes, en présence d'une résine échangeuse de cation qui est à base de copolymère de styrène-divinylbenzène, et présente une teneur en divinylbenzène allant de 8 à 18% et une dimension particulaire comprise entre 0,3 et 1,5 mm, à titre de catalyseur, procédé dans lequel l'oléfine à l'état gazeux (phase gazeuse) et l'acide carboxylique en tant que phase liquide s'écoulent parallèlement à travers une tube transversal de réaction contenant le catalyseur, lequel procédé est caractérisé en ce que:

a) avant de commencer la réaction, le catalyseur est soumis à un traitement de déshydratation suivi d'un traitement d'immersion dans l'acide carboxylique;

b) l'acide carboxylique liquide et l'oléfine gazeuse sont alors alimentés concurremment à travers le fond de l'enceinte de réaction, le catalyseur étant pendant toute la réaction complètement inondé d'acide carboxylique; et

c) la réaction est effectuée lorsque l'oléfine utilisée est le propylène, à une pression de 6 à 20 $kg/cm^2$, et lorsque l'oléfine utilisée est le butylène, à une pression d'environ 3,5 $kg/cm^2$, avec des températures de réaction comprises entre 70 et 115°C.

2. Procédé selon la revendication 1, caractérisé en ce que l'acide carboxylique est introduit avec une vitesse spatiale horaire liquide (VSHL) de 2,00 à 1,5 $h^{-1}$.

3. Procédé selon la revendication 2, caractérisé en ce que l'acide carboxylique et l'oléfine sont apportés dans un rapport molaire de 1:1 à 1:2,5.

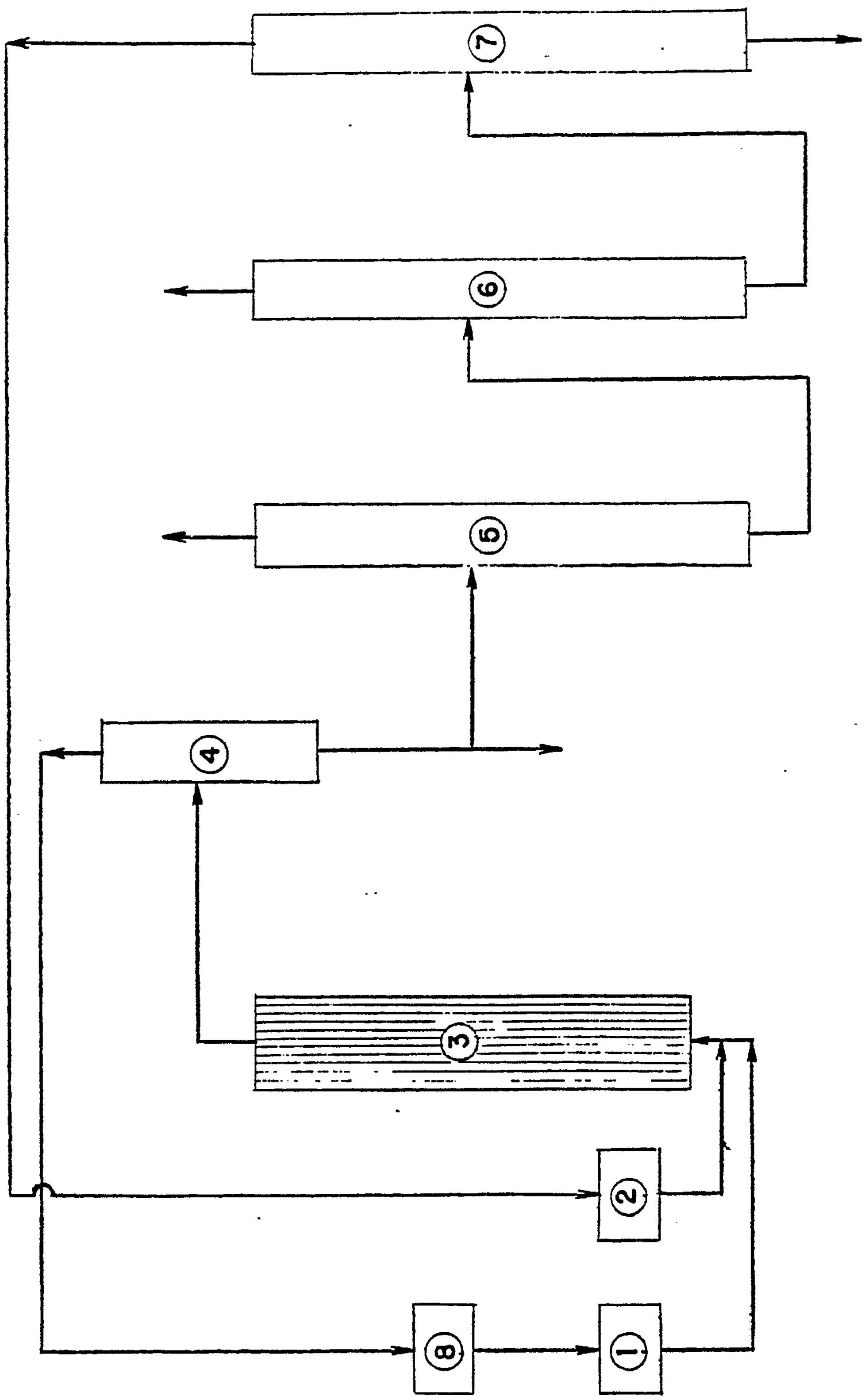

7
6
5
4
3
2
8
1